# EUROPEAN PATENT SPECIFICATION

(11) **EP 1 246 662 B1**
(45) Date of publication and mention of the grant of the patent: **31.08.2005**
(21) Application number: 00984484.6
(22) Date of filing: 13.12.2000
(51) Int. Cl.: A61M 39/02, A61K 9/00

(54) **OSMOTIC BENEFICIAL AGENT DELIVERY SYSTEM**
OSMOTISCHES SYSTEM ZUR WIRKSTOFFVERABREICHUNG
SYSTEME D'APPORT OSMOTIQUE D'AGENT BENEFIQUE

(30) Priority: 27.12.1999 US 171647 P
(43) Date of publication of application: 09.10.2002
(73) Proprietor: ALZA CORPORATION, Mountain View, CA 94039-7210 (US)
(72) Inventor: FERREIRA, Pamela, Santa Clara, CA 95051 (US); DAVIS, Craig, R., Newark, CA 94560 (US); BERRY, Stephen, A., Hollister, CA 95023 (US); STEWART, Gregory, R., Marlborough Massachusets 01752 (US); MAGRUDER, Judy, A., Mountain View, CA 94040 (US); LAU, Li-Ming, Palo Alto, CA 94301 (US); MAGRUDER, Paul, R., Mountain View, CA 94040 (US); HARRISON, Juan, M., San Francisco, CA 94118 (US); ROORDA, Wouter, California 94306 (US)
(74) Representative: Williams, Paul Edwin
(86) International application number: PCT/US2000/034315
(87) International publication number: WO 2001/047595

(56) References cited:
- WO-A-00/53253
- WO-A-98/42317
- US-A- 4 217 894
- US-A- 5 487 739

## Description

### BACKGROUND OF THE INVENTION

### Field of the Invention

The invention relates to osmotic beneficial agent delivery systems, and more particularly, the invention relates to an osmotic beneficial agent delivery system including an implant and a catheter for delivery of beneficial agents from the implant to a delivery site.

### Brief Description of the Related Art

Osmotic delivery devices such as those described in U.S. Patent Nos. 4,111,202; 4,111,203; and 4,203,439 can deliver a beneficial agent at a controlled delivery rate over an extended period of time. Osmotic delivery systems are very reliable in delivering a beneficial agent over an extended period of time called an administration period. In general osmotic delivery systems operate by imbibing fluid from an outside environment into the delivery system and releasing corresponding amounts of a beneficial agent from the delivery system. The osmotic delivery systems are generally implanted in tissue which provides the fluid environment from which fluid is drawn into the osmotic delivery system.

Osmotic delivery systems, commonly referred to as "osmotic pumps," generally include some type of capsule having one or more walls which selectively pass water into the interior of the capsule containing a water attracting agent. The absorption of water by the water attracting agent within the capsule reservoir creates an osmotic pressure within the capsule which causes a beneficial agent within the capsule to be delivered. The water attracting agent may be the beneficial agent being delivered to the patient, however, in most cases, a separate agent is used specifically for its ability to draw water into the capsule.

When a separate osmotic agent is used, the osmotic agent may be separated from the beneficial agent within the capsule by a movable dividing member, such as a membrane or a piston. The structure of the capsule is generally rigid such that as the osmotic agent takes in water from the environment and expands, the capsule does not expand. As the osmotic agent expands, the agent causes the movable dividing member to move, discharging the beneficial agent through an orifice or exit passage of the capsule. The beneficial agent is discharged through the exit passage at the same volumetric rate that water enters the osmotic agent through the semipermeable walls of the capsule.

The rate at which the beneficial agent is discharged from the delivery device is determined by many factors including the type of osmotic agent, the permeability of the semipermeable membrane, and the size and shape of the exit passage. The beneficial agent can be delivered at a controlled rate over periods of time which may be as long as a year.

One type of known osmotic delivery system includes a cylindrical capsule having an osmotic tablet such as salt separated by a piston from a beneficial agent reservoir inside the capsule. A first open end of the capsule is provided with a membrane plug to provide a semipermeable wall. The membrane plug seals the interior of the capsule from the exterior environment permitting only certain liquid molecules from the environment to permeate through the membrane plug into the osmotic agent chamber of the capsule. An opposite end of the capsule includes a delivery orifice through which the beneficial agent is delivered at a controlled delivery rate.

WO 98/42317A discloses a beneficial agent delivery system comprising an implant including an implant housing having a proximal end and a distal end, an osmotic agent contained within the housing, a beneficial agent reservoir within the housing, a fluid permeable membrane positioned in the proximal end of the housing which allows moisture to enter the housing and cause the osmotic agent with the housing to swell, and a fluid outlet at the distal end of the housing for dispensing the beneficial agent, and a catheter connected to the implant with an inlet of the catheter arranged to receive the beneficial agent dispensed from the fluid outlet of the implant.

It would be desirable to provide an osmotic delivery system including an implant containing the beneficial agent which is implanted at one location and a catheter connected to the implant which delivers the beneficial agent to another location. It also would be desirable to provide such an osmotic delivery system which delivers the beneficial agent over an extended period. It would also be desirable to provide and implantable system which allows delivery of a beneficial agent through a catheter to a treatment site, intravenously, or throughout a treatment region.

### SUMMARY OF THE INVENTION

The present invention relates to an osmotic beneficial agent delivery system including an implantable osmotic delivery device and a catheter for delivering the beneficial agent from the delivery device to a delivery location.

According to the present invention, a beneficial agent delivery system includes, an implant, an implantable docking station, and a catheter. According to an embodiment of the present invention the implant includes an implant housing having a proximal end and a distal end, an osmotic agent contained within the housing, a beneficial agent reservoir within the housing, a fluid permeable membrane positioned in the proximal end of the housing which allows moisture to enter the housing and cause the osmotic agent within the housing to swell, and a fluid outlet at the distal end of the housing for dispensing the beneficial agent. The implantable docking station is configured to receive the distal end of the implant housing. The catheter is connected to the implantable docking station with an inlet of the catheter arranged to receive the beneficial agent dispensed from the fluid outlet of the implant when the implant is received in the docking station.

According to another embodiment of the present invention, a beneficial agent delivery system includes a substantially cylindrical implant containing a beneficial agent, an implantable tubular docking station arranged to removably receive the implant, and a catheter connected to the docking station and arranged to receive the beneficial agent from the implant and dispense the beneficial agent to a treatment site when the implant is received in the docking station.

The present invention provides advantages of reliable delivery of a beneficial agent to delivery site over an extended administration period in a safe and convenient manner.

### BRIEF DESCRIPTION OF THE DRAWINGS

The invention will now be described in greater detail with reference to the preferred embodiments illustrated in the accompanying drawings, in which like elements bear like reference numerals, and wherein:
FIG. 1 is a side view of a first embodiment of a beneficial agent delivery system including an implant, a docking station, and a catheter;
FIG. 2 is a side view of the beneficial agent delivery system of FIG. 1 with the implant positioned in the docking station;
FIG. 3 is a schematic side cross-sectional view of the beneficial agent delivery system of FIG. 1 with the implant positioned in the docking station;
FIG. 4 is a side view of a second embodiment of a beneficial agent delivery system including an implant, a docking station, and inner and outer catheters;
FIG. 5 is a side view of the beneficial agent delivery system of FIG. 4 with the implant positioned in the docking station;
FIG. 6 is a side view of the beneficial agent delivery system of FIG. 1 with a multi-port catheter; and
FIG. 7 is a side view of a third embodiment of a beneficial agent delivery system including an implant, a docking station, and a catheter.

### DETAILED DESCRIPTION OF THE PREFERRED EMBODIMENTS

For many medical and therapeutic treatments it would be desirable to provide an implanted agent delivery catheter positioned to deliver a beneficial agent to a delivery site and a replaceable beneficial agent delivery device removably connectable to the catheter. The beneficial agent delivery system according to the present invention includes a catheter and docking station which are implanted in a patient with a distal end of the catheter positioned at the delivery site. The catheter and docking station are left in place while an implant containing the beneficial agent is removably connected to the catheter at the docking station and can be replaced as needed. The docking station provides a connection between the catheter and the implant and allows the implant to be replaced periodically while the catheter and docking station remain in place.

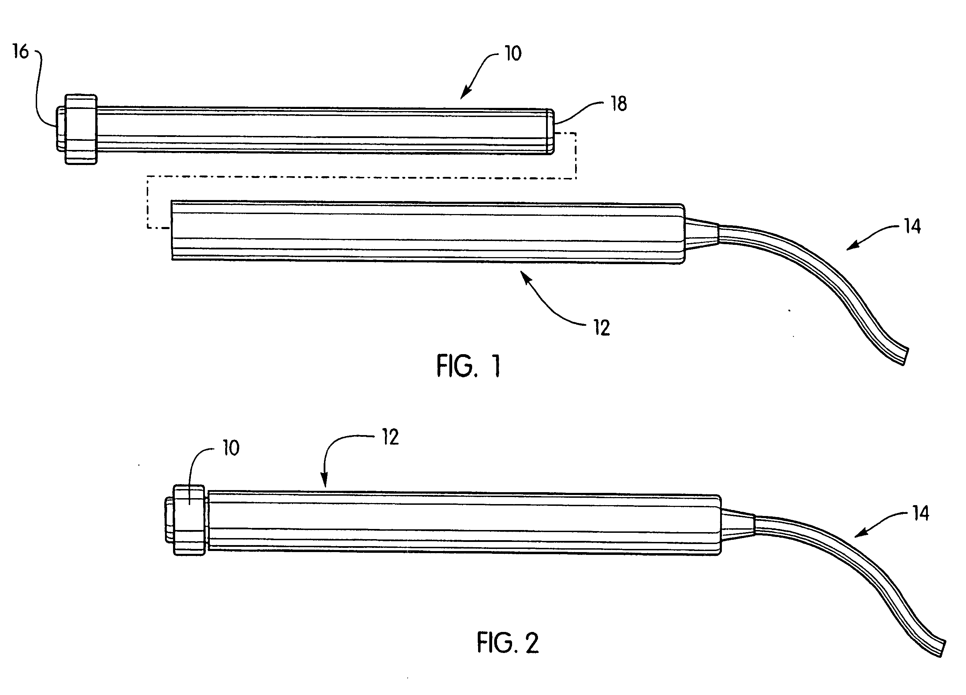

FIGS. 1 and 2 illustrate a beneficial agent delivery system including an implant 10, a docking station 12, and a catheter 14. The implant 10 is any known implant for controlled delivery of a beneficial agent. Preferably, the implant 10 is an osmotic beneficial agent delivery device having a proximal end 16 with a rate controlling membrane and a distal end 18 with a beneficial agent delivery orifice. The distal end 18 of the implant 10 is inserted into the tubular docking station 12 and allows the beneficial agent to be pumped from the implant into the catheter 14 for delivery to a delivery site. FIG. 2 illustrates the implant 10 inserted in the docking station.

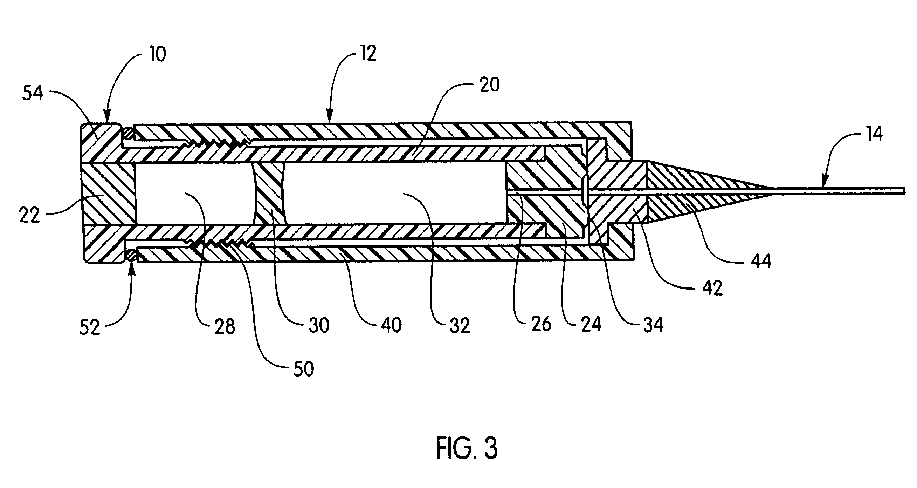

FIG. 3 is a schematic representation of one embodiment of a beneficial agent delivery system. The implant 10 includes a substantially cylindrical capsule 20, a rate controlling membrane 22, and a distal end cap 24 with a beneficial agent delivery orifice 26. The implant 10 may be of the type having an osmotic agent reservoir 28 and a beneficial agent reservoir 32 separated by a movable piston 30. In this type of implant 10, the structure of the capsule 20 is generally rigid such that as the osmotic agent takes in water from the environment and expands, the capsule does not expand. As the osmotic agent expands, the osmotic agent causes the movable piston 30 to move, discharging the beneficial agent through the beneficial agent delivery orifice 26. The beneficial agent is discharged through the orifice 26 at the same volumetric rate that water enters the osmotic agent through the semipermeable walls of the membrane 22.

The docking station 12 according to the exemplary embodiment of FIG. 3 includes a substantially cylindrical outer case 40. The docking station case 40 according to this embodiment extends over at least ½ of the length of the implant 10, and preferably over substantially the entire length of the implant. The catheter 14 is connected to the docking station 12 by a catheter hub 42 and strain relief portion 44. The docking station 12 may be fixed to the catheter 14 or to the catheter hub 42 by an adhesive or other attachment mechanism. For example, for a metal catheter, the catheter 14 may be connected to the docking station 12 by welding or brazing.

The distal end cap 24 of the implant 10 includes an annular ridge 34 on the distal end. This annular ridge 34 forms a fluid tight seal between the implant 10 and the docking station 12. The seal can be improved by forming the annular ridge 34 and/or the catheter hub of a resilient material. Alternative sealing mechanisms may also be provided, such as, an annular ridge on the catheter hub, or an annular seal between the exterior of the implant capsule 20 and the interior of the outer case 40.

As shown in FIG. 3, a threaded connection 50 is provided between the implant capsule 20 and the docking station 12 to retain the implant securely in the docking station. The threaded connection 50 may be replaced with any other known connection system, such as a snap-fit connection, a mechanical locking mechanism, or the like.

A flange 54 is preferably provided on the implant 10 which can be grasped during the implant exchange procedure. The system is preferably provided with a seal, such as the O-ring 52 which provides a seal between a distal end of the docking station outer case 40 and the flange 54. The O-ring 52 prevents fluid from entering a space between the implant 10 and the outer case 40. This O-ring 52 also can be used to prevent any beneficial agent from passing out of the system between the implant 10 and the docking station 12.

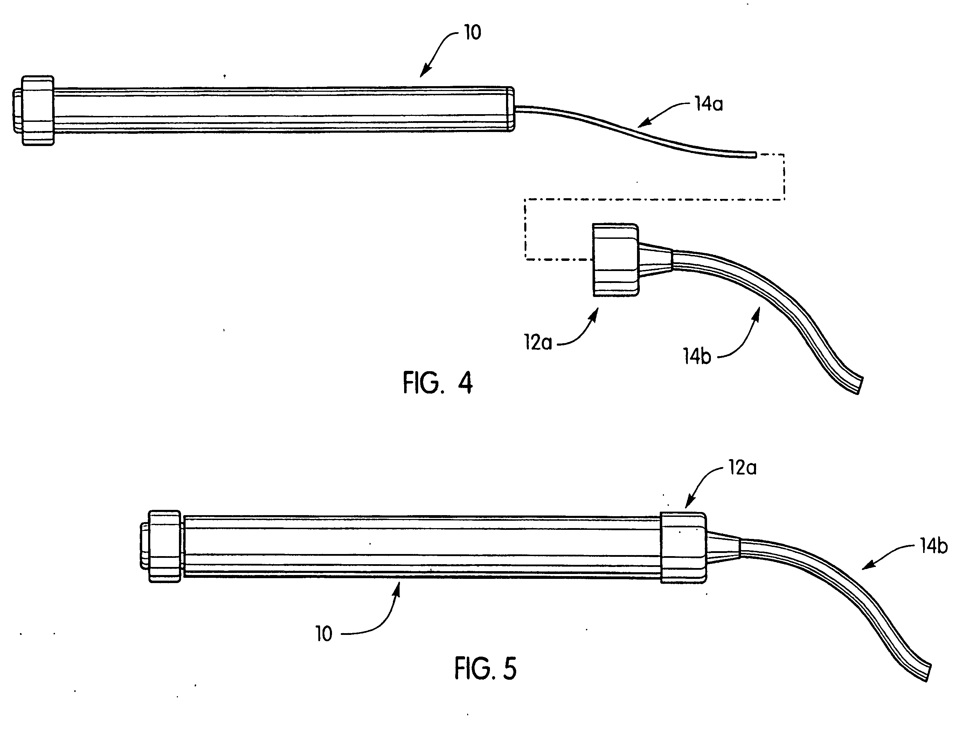

FIGS. 4 and 5 illustrate an alternative embodiment of the beneficial agent delivery system including an implant 10, a docking station 12a, an inner catheter 14a, and an outer catheter 14b. According to this embodiment, the docking station 12a is in the form of a distal end cap received over the distal end of the implant 10. The docking station 12a extends over only the distal end of the implant 10. The inner catheter 14a is connected to the distal end of the implant 10 and is replaceable with the implant. The outer catheter 14b is connected to the docking station 12a and has an inner lumen which is sized to receive the inner catheter 14a. The outer catheter 14b and the docking station 12a are implanted with a distal end of the outer catheter being placed accurately at the site of intended delivery of the beneficial agent.

The small inner catheter 14a has the advantage that the small lumen of this catheter can be used to delivery the beneficial agent at a very slow flow rate. The inner catheter 14a preferably has a lumen diameter of 0.2 mm or less. This small lumen catheter accommodates an implant with a slow flow rate, such as 100 ml per day or slower, and preferably 0.3 to 8 ml per day. The inner catheter 14a can also be replaced as a unit with the implant 10 and the outer catheter 14b can be flushed or used to draw blood when the implant is removed. Additionally, the inner catheter 14a can be formed of materials which are impermeable to the beneficial agent but does not necessarily have to be formed of a biocompatible material because it is shielded by the outer catheter 14b. Meanwhile, the outer catheter 14b can be formed of a biocompatible material, such as polyurethane, but does not need to be impermeable to the beneficial agent.

Preferably, there is as little space as possible between the inner and outer catheters 14a, 14b to prevent fluid entrapment. The inner catheter 14a may have a length which is the same as or different from the length of the outer catheter 14b. For example, the distal end of the inner catheter 14a may extend beyond the distal end of the outer catheter 14b and may have a plurality of holes for beneficial agent delivery over a treatment area.

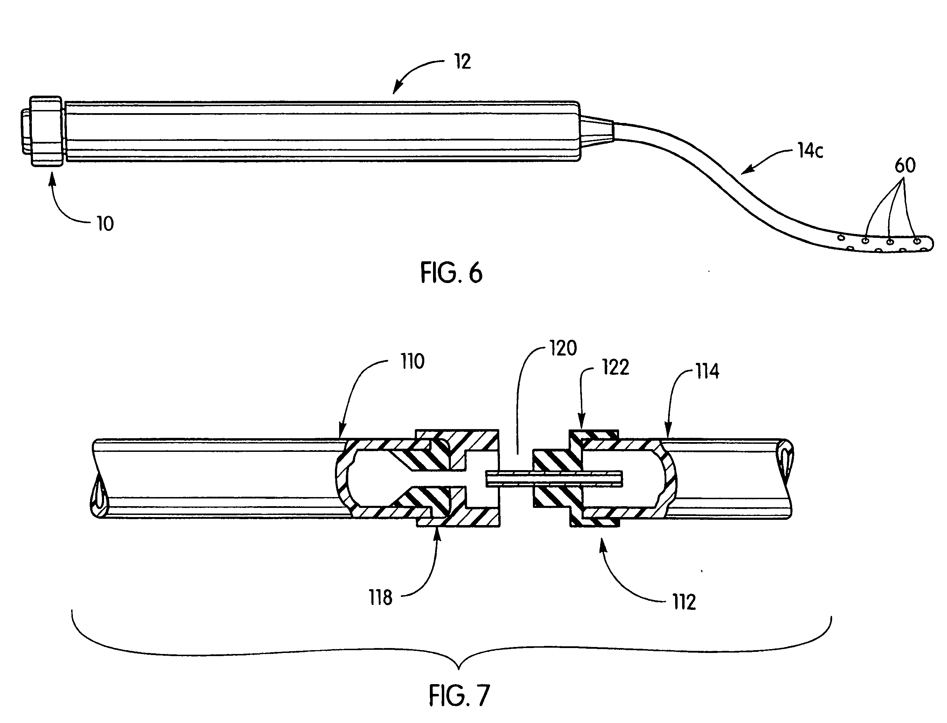

FIG. 6 illustrates an alternative embodiment of a beneficial agent delivery system for delivery of a beneficial agent throughout a targeted tissue site rather than at a single point. The system of FIG. 6 is substantially the same as the system shown in FIGS. 1 and 2 except that the distal end of the catheter 14c includes a plurality of holes 60. The holes 60 allow delivery of the beneficial agent over an area or region of the body. The arrangement, number, and size of the holes 60 will vary depending on the delivery area and pattern desired.

The system of FIG. 6 is particularly useful for medical applications in which it is desirable to deliver a beneficial agent over an area or region of the body. For example, in bone repair it may be more efficacious to deliver a drug to the entire tissue area between two fracture points. The region between the bone at a fracture point would be saturated with the drug to encourage bone reformation throughout the region. According to one preferred embodiment of the invention, the catheter 14c or at least the distal tip of the catheter is formed of a bioerodible material, such as PLGA, so that removal of the catheter is not required.

FIG. 7 illustrates an alternative embodiment of system for connecting a catheter 114 to an implant 110 with a docking station 112. According to this embodiment, the implant 110 is provided with a connector 118 in the form of a flow guide which is attached to the distal end of the implant. The connector 118 may be attached to the implant 110 by any known mechanical connecting method, such as adhesive, crimping, threading, welding, and the like. At the proximal end of the catheter 114, an alignment tube 120 is threaded into or otherwise attached to the catheter. An outer casing 122 is attached to the alignment tube 120 and secured to the proximal end of the catheter 114. The outer casing 122 is connected to the catheter 114 by any known mechanical connecting method, such as adhesive, crimping, threading, welding, and the like. The alignment tube 120 and the outer casing 122 form the docking station 112 for removably attaching the implant 110 to the catheter 114.

The implant 110 according to the embodiment of FIG. 7 is replaceably connected to the docking station 112 by inserting the alignment tube 120 into the connector 118. The implant 110 is preferably secured in the docking station by a locking mechanism (not shown) which may be a threaded nut, a clamp, or other mechanical fastener.

In operation of all of the embodiments of the present invention, the docking station 12 and catheter 14 are implanted and may be left in place for an extended period of time, preferably many years. The implant 10 will be implanted, and removed and replaced periodically. The time period for replacement will vary depending on the payload and delivery rate of the implant 10. The removal and replacement of the implant 10 from the docking station 12 may be done by hand or with the assistance of specially designed tools. Although the implant 10, docking station 12, and catheter 14 are all preferably implanted within the body of a patient, the system may also be used with the docking station and implant positioned outside of the body.

One example of an application for the present invention is for the intravenous delivery of factor VIII for treatment of hemophilia A. The catheter is implanted into a patient with a distal end of the catheter positioned in a blood vessel. The implant containing the factor VIII is connected to the catheter by the docking station for intravenous delivery. The implant is replaced on a regular basis as long as treatment is continued. The treatment can also be changed by changing the implant.

Other uses of the delivery system of the present invention include intrapericadial, intrathecal, inner ear, vascular, and other treatments. For example, anti-athersclerotic agents can be delivered for treatment of coronary artery disease, anti-thrombotic agents can be delivered to reduce restenosis, opiates (fentanyl or sufentanil) can be delivered for treatment of chronic malignant pain, baclofen can be delivered for treatment of spasticity (spinal, cerebral palsy), chemotherapy agents can be delivered to the inner ear and other organs, gentamicin can be delivered to the inner ear to treat tinnitus, and anti-thrombotic agents (such as heparin) can be delivered to vascular grafts to promote graft patency.

Examples of semipermeable materials for the membrane plug 22 include, but are not limited to, polyurethane, polyetherblockamide (PEBAX, commercially available from ELF ATOCHEM, Inc.), injection-moldable thermoplastic polymers with some hydrophilicity such as ethylene vinyl alcohol (EVA), and hydrophilic acrylate polymers, such as hydroxyethyl methacrylate (HEMA). In general, the membrane plug 30 is made from semipermeable materials having a water uptake ranging from 1% to 80%, and preferably less than 50%. The composition of the semipermeable membrane plug 22 is permeable to the passage of external liquids such as water and biological liquids, and it is substantially impermeable to the passage of beneficial agents, osmopolymers, osmagents, and the like.

Other materials for the membrane plug 22 are hytrel polyester elastomers (DuPont), cellulose esters, cellulose ethers and cellulose ester-ethers, water flux enhanced ethylene-vinyl acetate copolymers, semipermeable membranes made by blending a rigid polymer with water-soluble low molecular weight compounds, and other semipermeable materials well known in the art. The above cellulosic polymers have a degree of substitution, D.S., on the anhydroglucose unit, from greater than 0 up to 3 inclusive. "Degree of substitution" or "D.S." means the average number of hydroxyl groups originally present on the anhydroglucose unit comprising the cellulose polymer that are replaced by a substituting group. Representative materials include, but are not limited to, one selected from the group consisting of cellulose acylate, cellulose diacylate, cellulose triacylate, cellulose acetate, cellulose diacetate, cellulose triacetate, mono-, di-, and tricellulose alkanylates, mono-, di-, and tricellulose aroylates, and the like. Exemplary cellulosic polymers include cellulose acetate having a D.S. up to 1 and an acetyl content up to 21 %; cellulose acetate having a D.S. of 1 to 2 and an acetyl content of 21 % to 35 %; cellulose acetate having a D.S. of 2 to 3 and an acetyl content of 35 % to 44.8%, and the like. More specific cellulosic polymers include cellulose propionate having a D. S. of 1.8 and a propionyl content of 39.2 % to 45 % and a hydroxyl content of 2.8% to 5.4%; cellulose acetate butyrate having a D.S. of 1.8 and an acetyl content of 13 % to 15 % and a butyryl content of 34 % to 39%; cellulose acetate butyrate having an acetyl content of 2% to 29%, a butyryl content of 17 % to 53 % and a hydroxyl content of 0.5% to 4.7%; cellulose acetate butyrate having a D.S. of 1.8, and acetyl content of 4% average weight percent and a butyryl content of 51 %; cellulose triacylates having a D.S. of 2.9 to 3 such as cellulose trivalerate, cellulose trilaurate, cellulose tripalmitate, cellulose trisuccinate, and cellulose trioctanoate; cellulose diacylates having a D.S. of 2.2 to 2.6 such as cellulose disuccinate, cellulose dipalmitate, cellulose dioctanoate, cellulose dipentate; coesters of cellulose such as cellulose acetate butyrate and cellulose, cellulose acetate propionate, and the like.

Materials which may be used for the capsule 20 and the outer case 40 should be sufficiently strong to ensure that the capsule and outer case will not leak, crack, break, or distort under stresses to which they would be subjected during implanting or under stresses due to the pressures generated during operation. The capsule 20 and outer case 40 may be formed of chemically inert and biocompatible, natural or synthetic materials which are known in the art. The material of the capsule 20 and outer case 40 is preferably a non-bioerodible material which remains in the patient after use, such as titanium. However, the material of the capsule may alternatively be of bioerodible material which bioerodes in the environment after dispensing of the beneficial agent. Generally, preferred materials for the capsule are those acceptable for human implants.

In general, typical materials of construction suitable for the capsule 20 and outer case 40 according to the present invention include non-reactive polymers or biocompatible metals or alloys. The polymers include acrylonitrile polymers such as acrylonitrile-butadiene-styrene terpolymer, and the like; halogenated polymers such as polytetraflouroethylene, polychlorotrifluoroethylene, copolymer tetrafluoroethylene and hexafluoropropylene; polyimide; polysulfone; polycarbonate; polyethylene; polypropylene; polyvinylchloride-acrylic copolymer; polycarbonate-acrylonitrile-butadiene-styrene; polystyrene; and the like. Metallic materials useful for the capsule include stainless steel, titanium, platinum, tantalum, gold, and their alloys, as well as gold-plated ferrous alloys, platinum-plated ferrous alloys, cobalt-chromium alloys and titanium nitride coated stainless steel.

In general, materials suitable for use in the piston 30 are elastomeric materials including the non-reactive polymers listed above, as well as elastomers in general, such as polyurethanes and polyamides, chlorinated rubbers, styrenebutadiene rubbers, and chloroprene rubbers.

The osmotic tablet is an osmotic agent which is a fluid-attracting agent used to drive the flow of the beneficial agent. The osmotic agent may be an osmagent, an osmopolymer, or a mixture of the two. Species which fall within the category of osmagent, i.e., the non-volatile species which are soluble in water and create the osmotic gradient driving the osmotic inflow of water, vary widely. Examples are well known in the art and include magnesium sulfate, magnesium chloride, potassium sulfate, sodium chloride, sodium sulfate, lithium sulfate, sodium phosphate, potassium phosphate, d-mannitol, sorbitol, inositol, urea, magnesium succinate, tartaric acid, raffinose, and various monosaccharides, oligosaccharides and polysaccharides such as sucrose, glucose, lactose, fructose, and dextran, as well as mixtures of any of these various species.

Species which fall within the category of osmopolymer are hydrophilic polymers that swell upon contact with water, and these vary widely as well. Osmopolymers may be of plant or animal origin, or synthetic, and examples of osmopolymers are well known in the art. Examples include: poly(hydroxy-alkyl methacrylates) with molecular weight of 30,000 to 5,000,000, poly(vinylpyrrolidone) with molecular weight of 10,000 to 360,000, anionic and cationic hydrogels, polyelectrolyte complexes, poly(vinyl alcohol) having low acetate residual, optionally cross-linked with glyoxal, formaldehyde or glutaraldehyde and having a degree of polymerization of 200 to 30,000, a mixture of methyl cellulose, cross-linked agar and carboxymethylcellulose, a mixture of hydroxypropyl methylcellulose and sodium carboxymethylcellulose, polymers of N-vinyllactams, polyoxyethylene-polyoxypropylene gels, polyoxybutylene-polyethylene block copolymer gels, carob gum, polyacrylic gels, polyester gels, polyurea gels, polyether gels, polyamide gels, polypeptide gels, polyamino acid gels, polycellulosic gels, carbopol acidic carboxy polymers having molecular weights of 250,000 to 4,000,000, Cyanamer polyacrylamides, cross-linked indene-maleic anhydride polymers, Good-Rite polyacrylic acids having molecular weights of 80,000 to 200,000, Polyox polyethylene oxide polymers having molecular weights of 100,000 to 5,000,000, starch graft copolymers, and Aqua-Keeps acrylate polymer polysaccharides.

Although the present invention has been described with respect to an osmotic system having as osmotic agent and a beneficial agent, it should be understood that the osmotic agent may be incorporated into the beneficial agent.

In one embodiment of the invention, the beneficial agents contained in the beneficial agent reservoir 32 are flowable compositions such as liquids, suspension, slurries, pastes, or powders and are poured into the capsule 20 prior to insertion of the membrane plug 22. Alternatively, such flowable compositions may be injected with a needle through a delivery port or membrane plug, which allows for filling without air bubbles. Still further alternatives may include any of the wide variety of techniques known in the art for forming capsules used in the pharmaceutical industry.

Animals to whom drugs may be administered using systems of this invention include humans and other animals. The invention is of particular interest for application to humans and household, sport, and farm animals, particularly mammals.

The present invention applies to the administration of beneficial agents in general, which include any physiologically or pharmacologically active substance. The beneficial agent may be any of the agents which are known to be delivered to the body of a human or an animal such as drug agents, medicaments, vitamins, nutrients, or the like.

The beneficial agent can be present in this invention in a wide variety of chemical and physical forms, such as solids, liquids and slurries. On the molecular level, the various forms may include uncharged molecules, molecular complexes, and pharmaceutically acceptable acid addition and base addition salts such as hydrochlorides, hydrobromides, sulfate, laurylate, oleate, and salicylate. For acidic compounds, salts of metals, amines or organic cations may be used. Derivatives such as esters, ethers and amides can also be used. An active agent can be used alone or mixed with other active agents.

According to other embodiments of the present invention, the implant 10 may take different forms. For example, the piston 30 may be replaced with a member such as a diaphragm, partition, pad, flat sheet, spheroid, or rigid metal alloy, and may be made of any number of inert materials. Furthermore, the osmotic device may function without the piston 30, having simply an interface between the osmotic agent/fluid additive and the beneficial agent or having the osmotic agent incorporated in the beneficial agent.

While the invention has been described in detail with reference to the preferred embodiments thereof, it will be apparent to one skilled in the art that various changes and modifications can be made and equivalents employed, without departing from the present invention.

## Claims

1. A beneficial agent delivery system comprising:
an implant (10) containing a beneficial agent;
an implantable docking station (12) arranged to receive the implant; and
a catheter (14) connected to the docking station and arranged to receive the beneficial agent from the implant and dispense the beneficial agent to a treatment site when the implant is received in the docking station.

2. A beneficial agent delivery system according to claim 1, wherein the implant is substantially cylindrical and the docking station is tubular and arranged to removably receive the implant.

3. A beneficial agent delivery system according claim 1 or 2, wherein the docking station extends over at least half the length of the implant.

4. A beneficial agent delivery system according to any preceding claim, further comprising a seal provided between the docking station and the implant.

5. A beneficial agent delivery system according to claim 4, wherein the seal includes a resilient annular ridge (34) on the implant and/or an O-ring (52).

6. A beneficial agent delivery system according to any preceding claim, wherein the catheter has an inner diameter of 0.2 mm or less.

7. A beneficial agent delivery system according to any preceding claim, wherein the implant is configured to deliver the beneficial agent at flow rates of 100 ml per day or less.

8. A beneficial agent delivery system according to any preceding claim, wherein the catheter has a distal tip (14c) with a plurality of agent delivery holes (60).

9. A beneficial agent delivery system according to any preceding claim, wherein the catheter is bioerodable.

10. A beneficial agent delivery system according to any preceding claim, wherein the implant is an osmotic implant.

11. A beneficial agent delivery system according to claim 10, wherein the implant includes an implant housing having a proximal end (16) and a distal end (18), an osmotic agent contained within the housing, a beneficial agent reservoir within the housing, a fluid permeable membrane (22) positioned in the proximal end of the housing which can allow moisture to enter the housing and cause the osmotic agent within the housing to swell, and a fluid outlet at the distal end of the housing for dispensing the beneficial agent;
the implantable docking station is arranged to receive the distal end of the implant housing; and
the catheter connected to the implantable docking station has an inlet arranged to receive the beneficial agent dispensed from the fluid outlet of the implant when the implant is received in the docking station.

12. A beneficial agent delivery system according to claim 11, wherein the docking station is a tubular member which can receive the distal end of the implant housing inside the tubular member.

13. A beneficial agent delivery system according to any preceding claim, further comprising an inner catheter (14a) connected to a beneficial agent delivery orifice of the implant, wherein the inner catheter fits within the catheter connected to the docking station when the implant is connected to the docking station.

## Patentansprüche

1. Abgabesystem für einen Wirkstoff, umfassend:
ein Implantat (10), enthaltend einen Wirkstoff:
eine implantierbare Andock-Station (12), eingerichtet, um das Implantat aufzunehmen; und
einen Katheter (14), verbunden mit der Andock-Station und eingerichtet, um den Wirkstoff vom Implantat aufzunehmen und den Wirkstoff an einer Behandlungsstelle abzugeben, wenn das Implantat in der Andock-Station aufgenommen ist.

2. Abgabesystem für einen Wirkstoff nach Anspruch 1, worin das Implantat im Wesentlichen zylindrisch ist und die Andock-Station röhrenförmig ist und eingerichtet ist, um das Implantat entfernbar aufzunehmen.

3. Abgabesystem für einen Wirkstoff nach Anspruch 1 oder 2, worin sich die Andock-Station über mindestens die Hälfte der Länge des Implantats erstreckt.

4. Abgabesystem für einen Wirkstoff nach irgendeinem der vorangehenden Ansprüche, weiterhin umfassend eine Dichtung, vorgesehen zwischen der Andock-Station und dem Implantat.

5. Abgabesystem für einen Wirkstoff nach Anspruch 4, worin die Dichtung einen elastischen ringförmigen Grat (34) auf dem Implantat und/oder einen O-Ring (52) umfasst.

6. Abgabesystem für einen Wirkstoff nach irgendeinem der vorangehenden Ansprüche, worin der Katheter einen Innendurchmesser von 0,2 mm oder weniger aufweist.

7. Abgabesystem für einen Wirkstoff nach irgendeinem der vorangehenden Ansprüche, worin das Implantat eingestellt ist, um den Wirkstoff bei Flußgeschwindigkeiten von 100 ml pro Tag oder weniger freizusetzen.

8. Abgabesystem für einen Wirkstoff nach irgendeinem der vorangehenden Ansprüche, worin der Katheter eine distale Düse (14c) mit einer Vielzahl von Wirkstoffaustrittsöffnungen (60) aufweist.

9. Abgabesystem für einen Wirkstoff nach irgendeinem der vorangehenden Ansprüche, worin der Katheter bioabbaubar ist.

10. Abgabesystem für einen Wirkstoff nach irgendeinem der vorangehenden Ansprüche, worin das Implantat ein osmotisches Implantat ist.

11. Abgabesystem für einen Wirkstoff nach Anspruch 10, worin das Implantat ein Implantat-Gehäuse umfasst mit einem proximalen Ende (16) und einem distalen Ende (18), einem osmotischen Mittel, enthalten im Gehäuse, einem Reservoir für einen Wirkstoff im Gehäuse, einer flüssigkeitsdurchlässigen Membran (22), angeordnet am proximalen Ende des Gehäuses, die es ermöglichen kann, dass Feuchtigkeit in das Gehäuse eintritt und das osmotische Mittel im Gehäuse anschwellen lässt, und einen Flüssigkeitsauslass am distalen Ende des Gehäuses, um den Wirkstoff abzugeben:
die implantierbare Andock-Station eingerichtet ist, um das distale Ende des Implantat-Gehäuses aufzunehmen; und
der mit der implantierbaren Andock-Station verbundene Katheter einen Einlaß aufweist, der so eingerichtet ist, dass er den vom Flüssigkeitsauslass des Implantats abgegebenen Wirkstoff aufnimmt, wenn das Implantat in der Andock-Station aufgenommen ist.

12. Abgabesystem für einen Wirkstoff nach Anspruch 11, worin die Andock-Station ein röhrenförmiges Teil darstellt, das das distale Ende des Implantat-Gehäuses im röhrenförmigen Teil aufnehmen kann.

13. Abgabesystem für einen Wirkstoff nach irgendeinem der vorangehenden Ansprüche, ferner umfassend einen inneren Katheter (14a), verbunden mit einer Wirkstoffzuführöffnung des Implantats, worin der innere Katheter in den Katheter, der mit der Andock-Station verbunden ist, während das Implantat mit der Andock-Station verbunden ist, hineinpasst.

## Revendications

1. Système de fourniture d'un agent bénéfique, comprenant :
un implant (10) contenant un agent bénéfique ;
un réceptacle d'arrimage implantable (12), adapté pour recevoir l'implant ; et
un cathéter (14) connecté au réceptacle d'arrimage et adapté pour recevoir l'agent bénéfique depuis l'implant et pour délivrer l'agent bénéfique à un site de traitement lorsque l'implant est reçu dans le réceptacle d'arrimage.

2. Système de fourniture d'un agent bénéfique selon la revendication 1, dans lequel l'implant est substantiellement cylindrique et le réceptacle d'arrimage est tubulaire et est et adapté à recevoir l'implant de manière amovible.

3. Système de fourniture d'un agent bénéfique selon la revendication 1 ou la revendication 2, dans lequel le réceptacle d'arrimage s'étend sur au moins la moitié de la longueur de l'implant.

4. Système de fourniture d'un agent bénéfique selon l'une des revendications précédentes, comprenant en outre un moyen d'étanchéité aménagé entre le réceptacle d'arrimage et l'implant.

5. Système de fourniture d'un agent bénéfique selon la revendication 4, dans lequel le moyen d'étanchéité inclut une arête annuaire souple (34) sur l'implant et/ou un joint torique (52).

6. Système de fourniture d'un agent bénéfique selon l'une des revendications précédentes, dans lequel le cathéter a un diamètre interne de 0,2 mm ou moins.

7. Système de fourniture d'un agent bénéfique selon l'une des revendications précédentes, dans lequel l'implant est configuré de manière à fournir l'agent bénéfique à un débit de 100 ml par jour ou moins.

8. Système de fourniture d'un agent bénéfique selon l'une des revendications précédentes, dans lequel le cathéter a une extrémité distale (14c) comprenant une pluralité de trous (60) de délivrance de l'agent.

9. Système de fourniture d'un agent bénéfique selon l'une des revendications précédentes, dans lequel le cathéter est biorésorbable.

10. Système de fourniture d'un agent bénéfique selon l'une des revendications précédentes, dans lequel l'implant est un implant osmotique.

11. Système de fourniture d'un agent bénéfique selon la revendication 10, dans lequel l'implant comprend un logement d'implant ayant une extrémité proximale (16) et une extrémité distale (18), un agent osmotique contenu dans le logement, un réservoir d'agent bénéfique dans le logement, une membrane (22) perméable aux fluides positionnée dans l'extrémité proximale du logement, qui peut permettre à de l'humidité d'entrer dans le logement et d'amener l'agent osmotique situé dans le logement à gonfler, et un orifice de sortie de fluide à l'extrémité distale du logement pour délivrer l'agent bénéfique ;
le réceptacle d'arrimage implantable est adapté à recevoir l'extrémité distale du logement d'implant ; et
le cathéter connecté au réceptacle d'arrimage implantable comprend un orifice d'entrée adapté à recevoir l'agent bénéfique délivré depuis l'orifice de sortie de fluide de l'implant lorsque l'implant est reçu dans te réceptacle d'arrimage.

12. Système de fourniture d'un agent bénéfique selon la revendication 11, dans lequel le réceptacle d'arrimage est un organe tubulaire qui peut recevoir l'extrémité distale du logement d'implant à l'intérieur de l'organe tubulaire.

13. Système de fourniture d'un agent bénéfique selon l'une des revendications précédentes, comprenant en outre un cathéter interne (14a) connecté à l'orifice de délivrance d'un agent bénéfique de l'implant, dans lequel le cathéter intérieur s'adapte à l'intérieur du cathéter connecté au réceptacle d'arrimage lorsque l'implant est connecté au réceptacle d'arrimage.
